# EUROPEAN PATENT APPLICATION

(11) **EP 2 081 015 A2**
(43) Date of publication of application: **22.07.2009**
(21) Application number: 09150770.7
(22) Date of filing: 16.01.2009
(51) Int. Cl.: G01N 21/35, G01N 21/91

(54) **Apparatus for inspecting food**

(30) Priority: 18.01.2008 JP 2008009557
(71) Applicant: Sumitomo Electric Industries, Ltd., Osaka 541-0041 (JP)
(72) Inventor: Shimazu, Takayuki, Yokohama-shi, Kanagawa (JP); Katayama, Makoto, Yokohama-shi, Kanagawa (JP); Okuno, Toshiaki, Yokohama-shi, Kanagawa (JP); Tanaka, Masato, Yokohama-shi, Kanagawa (JP)
(74) Representative: Kreutzer, Ulrich

(57) **Abstract**

A food inspection apparatus 1 includes a light source or a plurality of light sources 10 that outputs near infrared light L1, and a detector unit 20 that measures a diffuse reflectance spectrum of light which is the near infrared light L1 and is diffusively reflected from an object 50 from among. The light source 10 and a detector unit 20 are arranged such that an angle defined by an optical path L2, along which the near infrared light is regularly reflected at a surface of the object, or an optical path L5, along which the near infrared light is transmitted through the object, and a straight line L3 or L6 connecting an irradiation position P1 or P2 on the surface of the object irradiated with the near infrared light and the detector unit 20, is 45 degrees or greater.

## Description

### Technical Field

The present invention relates to a food inspection apparatus that measures diffuse reflection light obtained by irradiating food as an object with near infrared light and evaluates quality of the object on the basis of the measured result.

### Background Art

Quality evaluation of food by detecting a foreign material and something unusual during processing of the food has been further important as consumers pay further attention to safety of the food. Various methods for detecting foreign materials and something unusual have been studied.

Japanese Unexamined Patent Application Publication No. 2004-301690 discloses a method of inspecting a foreign material contained in food by irradiating the food with visible light or near infrared light and detecting reflected light from the food. With this method of inspecting food, inspection accuracy may seriously vary depending on the type and condition of visible light or near infrared light with which an object is irradiated. For example, if a light source is improper, a noise component increases. This may degrade an S/N ratio and hence an identification error or an analysis error may occur. It is difficult to constantly carry out an inspection with high accuracy.

### Disclosure of the Invention

### Problems to be Solved by the Invention

An object of the present invention is to provide a food inspection apparatus that improves accuracy of quality evaluation.

### Means for Solving the Problems

To attain the object, an apparatus is provided that evaluates quality of food as an object. The apparatus includes (1) a light source or a plurality of light sources that outputs near infrared light, and (2) a detector unit that measures a diffuse reflectance spectrum of light which is the near infrared light output from the light source or the plurality of light sources and is diffusively reflected by the object. In the apparatus, an angle defined by an optical path, along which the near infrared light is regularly reflected at a surface of the object, or an optical path, along which the near infrared light is transmitted through the object, and a straight line connecting an irradiation position on the surface of the object irradiated with the near infrared light and the detector unit, is 45 degrees or greater.

### Brief Description of the Drawings

Figure 1 is a conceptual diagram showing a food inspection apparatus according to a first embodiment of the present invention;

Figure 2 is a conceptual diagram showing a food inspection apparatus according to a second embodiment of the present invention;

Figure 3 is a conceptual diagram showing a food inspection apparatus according to a third embodiment of the present invention;

Figure 4 is a graph showing KM absorbance second differentiation spectra when an angle a1 in Fig. 1 is 20 degrees;

Figure 5 is a graph showing KM absorbance second differentiation spectra when the angle a1 in Fig. 1 is 30 degrees;

Figure 6 is a graph showing KM absorbance second differentiation spectra when the angle a1 in Fig. 1 is 40 degrees;

Figure 7 is a graph showing KM absorbance second differentiation spectra when the angle a1 in Fig. 1 is 50 degrees;

Figure 8 is a graph showing KM absorbance second differentiation spectra when the angle a1 in Fig. 1 is 60 degrees;

Figure 9 is a graph showing KM absorbance second differentiation spectra when the angle a1 in Fig. 1 is 70 degrees; and

Figure 10 is a graph showing KM absorbance second differentiation spectra when the angle a1 in Fig. 1 is 80 degrees.

### Best Mode for Carrying Out the Invention

Embodiments of the present invention are described below with reference to figures. The figures are provided for description, and the scope of the invention should not be limited by the figures. In the figures, equivalent numerals refer equivalent components to avoid redundant description. The ratio of dimensions in the figures is not always accurate.

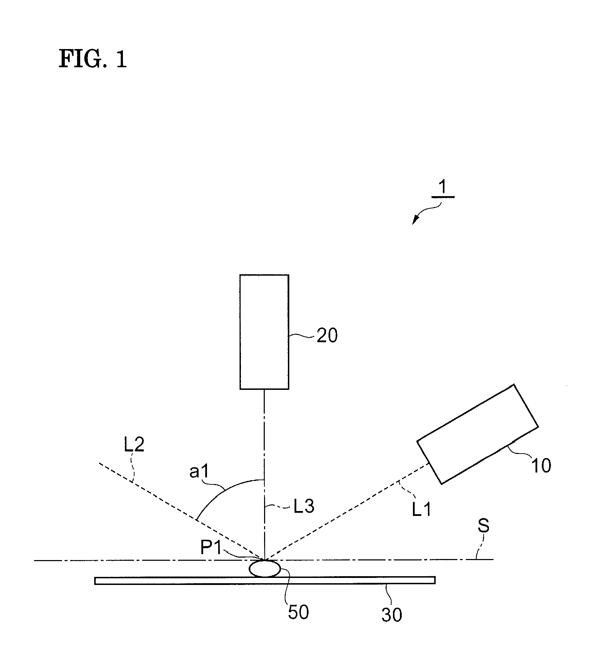

First Embodiment Figure 1 is a conceptual diagram showing a food inspection apparatus 1 according to a first embodiment of the present invention. The food inspection apparatus 1 includes a light source 10, a detector unit 20, and an inspection stage 30. The light source 10 irradiates an object 50 arranged on the inspection stage 30 with near infrared light. The detector unit 20 detects diffuse reflection light from the object 50.

The light source 10 outputs near infrared light with a predetermined wavelength band to an irradiation position P1 on a surface of the object 50. The light source 10 may be a halogen lamp. In this case, since the halogen lamp is inexpensive, a food inspection apparatus having higher versatility can be provided. Alternatively, the light source 10 may be an SC light source including a seed light source and a nonlinear medium. In the SC light source, light output from the seed light source is input to the nonlinear medium, the spectrum is widened by a nonlinear optical effect in the nonlinear medium, and the light is output as supercontinuum (SC) light. In this case, heating with the light source is reduced as compared with the case of the halogen lamp; accordingly, food which must not be heated or food necessary to be concerned with deterioration by heating may be applied to an object 50. Still alternatively, the light source 10 may be a laser light source that outputs near infrared light with a specific wavelength band (for example, wavelengths from 1000 to 2500 nm).

The light source 10 preferably has a function of modulating the intensity of the near infrared light to be output from the light source 10. In this case, the detector unit measures a diffuse reflectance spectrum of the diffuse reflection light in synchronization with a cycle of the intensity modulation of the light source. Accordingly, light other than the diffuse reflection light caused by the near infrared light output from the light source is prevented from being detected by the detector unit. Thus, the detector unit can detect the diffuse reflection light with a good S/N ratio. On the basis of the result, the quality of the food as the object can be correctly evaluated.

The detector unit 20 detects light as a diffuse reflectance spectrum, the light being the near infrared light which is output from the light source 10, diffusively reflected at the irradiation position P1 on the surface of the object 50, and then is output toward the detector unit 20. The detector unit 20 may be, for example, an MCT detector unit formed of mercury, cadmium, and tellurium; an InGaAs detector unit; etc.

The inspection stage 30 is a stage on which food serving as the object 50 is placed. The inspection stage 30 is preferably made of a material transmitting the near infrared light output from the light source 10. In the first embodiment, the detector unit 20 is perpendicular to an irradiation plane S which is a plane containing the irradiation position P1 and being parallel to the inspection stage 30.

In the first embodiment, the near infrared light output from the light source 10 is light with a wavelength ranging from 800 to 2500 nm. The measurement is preferably carried out with a wavelength ranging from 1000 to 2500 nm. However, the wavelength range may be properly changed depending on the purpose of use.

Here, the wavelength range of the near infrared light output from the light source 10 is described in more detail. The quality evaluation with the food inspection apparatus 1 includes detection of a foreign material contained in the object 50, and detection of something unusual of the object 50.

The foreign material may be a substance originated from a human body, such as a hair, a metal piece of equipment used for processing of the food, or an impurity of the food. Such a foreign material has an absorption band for the near infrared light in the above-described wavelength range. Hence, when the object 50 is irradiated with the near infrared light in the above-described wavelength range, specific absorption peaks caused by such a foreign material can be detected.

A method of detecting something unusual may be a method of measuring moisture and sugar contained in food. For example, sugar has absorption peaks around the wavelengths of 1500 and 2100 nm. Hence, at least near infrared light in a wavelength range of 1500±100 nm and near infrared light in a wavelength range of 2100±100 nm are output, the diffuse reflection light is measured, and the measurement result is analyzed. Accordingly, peaks caused by the sugar in the food can be detected. The type and content of the sugar can be obtained with reference to the positions and intensities of the peaks caused by the sugar, thereby enabling the quality evaluation of the food. Also, for example, moisture contained in the food has an absorption peak around the wavelength of 1450 nm. Hence, at least near infrared light in a wavelength range of 1450±100 nm is output to the object 50. The diffuse reflection light is measured, and thus the moisture content can be calculated on the basis of the height of the peak around the wavelength of 1450 nm included in the measured result.

Next, arrangement of the light source 10 and the detector unit 20 is described. Near infrared light L1 output from the light source 10 reaches the irradiation position P1 of the object 50. A plane containing the irradiation position P1 and being parallel to the inspection stage 30 defines an irradiation plane S. When the near infrared light L1 is regularly reflected with respect to the irradiation plane S, the regular reflection light propagates along an optical path L2. Meanwhile, light which is diffusively reflected at the irradiation position P1 and diffused along an optical path L3 reaches the detector unit 20. At this time, the light source 10 and the detector unit 20 are arranged such that an angle a1 is 45 degrees or greater, the angle a1 being defined by the optical path L3, which is a straight line connecting the detector unit 20 and the irradiation position P1, and the optical path L2, along which the near infrared light output from the light source 10 is regularly reflected.

In general, when an object, having a low transmittance for near infrared light, is irradiated with the near infrared light, regular reflection light and diffuse reflection light are generated. Like the food inspection apparatus 1, in a case of a food inspection apparatus that measures diffuse reflection light and evaluates the quality of an object on the basis of the measured result, properly detecting the diffuse reflection light is an important factor to improve evaluation accuracy.

Like the food inspection apparatus 1, since the angle a1 defined by the optical path L3 and the optical path L2 is 45 degrees or greater, regular reflection light can be effectively prevented from being incident on the detector unit 20 even when the object 50 has a rough shape at the irradiation position P1 of the near infrared light. Accordingly, the detector unit can measure a diffuse reflectance spectrum with higher accuracy, and hence, the food inspection apparatus 1 can carry out the quality evaluation with high accuracy

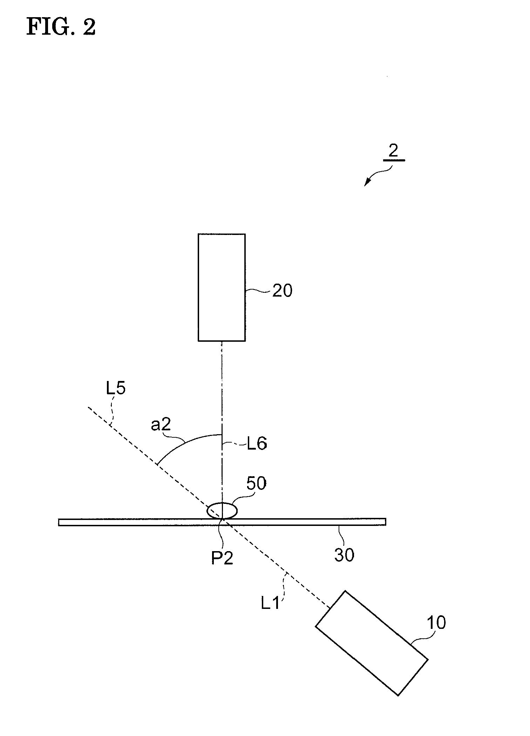

Second Embodiment Figure 2 is a conceptual diagram showing a food inspection apparatus 2 according to a second embodiment of the present invention. The food inspection apparatus 2 is different from the food inspection apparatus 1 according to the first embodiment in that light output from a light source 10 which locates under an inspection stage irradiates an object 50.

In the food inspection apparatus 2, near infrared light L1 is output from the light source 10 toward the object 50. The object is irradiated with the near infrared light L1 at an irradiation position P2. Light propagating along an optical path L6 of the near infrared light diffusively reflected at the irradiation position P2 reaches a detector unit 20.

At this time, the light source 10 and the detector unit 20 are arranged such that an angle a2 is 45 degrees or greater, the angle a2 being defined by the optical path L6, which is a straight line connecting the detector unit 20 and the irradiation position P2, and an optical path L5, along which the near infrared light L1 output from the light source 10 is transmitted through the object 50. Since the angle a2 is 45 degrees or greater, near infrared light which is not diffusively reflected by the object 50 but transmitted through the object 50 can be prevented from being incident on the detector unit 20. Accordingly, the detector unit can measure a diffuse reflectance spectrum with higher accuracy, and hence, the accuracy of the quality evaluation of food on the basis of the measurement of diffuse reflection light can be further improved.

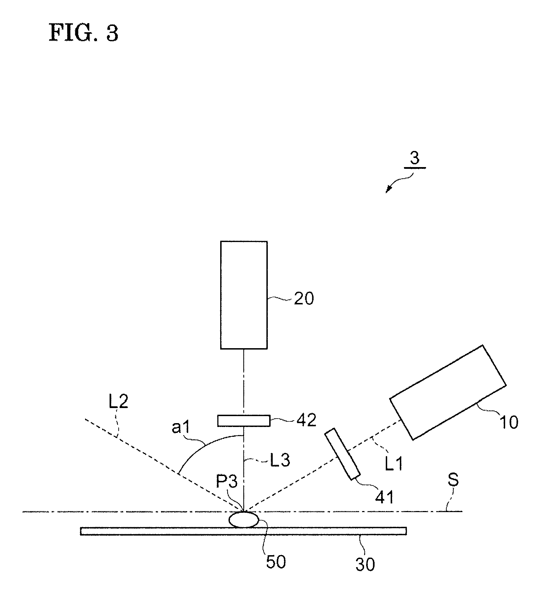

Third Embodiment Figure 3 is a conceptual diagram showing a food inspection apparatus 3 according to a third embodiment of the present invention. The food inspection apparatus 3 is different from the food inspection apparatus 1 according to the first embodiment in that polarizing plates 41 and 42 are provided between a light source 10 and an irradiation position P3 on an object 50, and between the irradiation position P3 and a detector unit 20. The polarizing plate 41 selectively transmits only linearly polarized light component in a specific direction of near infrared light L1 output from the light source 10, and the transmitted light reaches the irradiation position P3. The polarizing plate 42 transmits only linearly polarized light component in the same direction as that of the polarizing plate 41 from among light diffusively reflected at the irradiation position P3 along an optical path L3, and the transmitted light reaches the detector unit 20.

With the food inspection apparatus 3, the measurement accuracy of the diffuse reflection light is improved, and the accuracy of the quality evaluation of food can be further improved in a similar manner to the first embodiment. Further, since the food inspection apparatus 3 includes the two polarizing plates 41 and 42, light different from the diffuse reflection light of the near infrared light output from the light source 10 can be prevented from reaching the detector unit 20. This can further improve the accuracy of the quality evaluation of food by the measurement of the diffuse reflection light.

The present invention is not limited to the above-described embodiments, and various modifications may be made. For example, the two polarizing plates provided in the third embodiment may be applied to the second embodiment.

Also, a plurality of light sources may be arranged to output light to a common irradiation position. For example, when a plurality of light sources is arranged in the food inspection apparatus 1, each of the plurality of light sources preferably has an equivalent angle defined by a straight line (optical path L1 in Fig. 1) connecting the light source and the irradiation position P1, and an optical path (optical path L3 in Fig. 1) connecting the irradiation position P1 and the detector unit 20. Each adjacent light sources of the plurality of light sources preferably have an equivalent angle defined by straight lines connecting the adjacent light sources and the irradiation position P1. The plurality of light sources is preferably arranged at equivalent intervals around the straight line (optical path L3) connecting the irradiation position P1 and the detector unit 20.

Also, when a plurality of light sources is arranged in the food inspection apparatus 2 according to the second embodiment, each of the plurality of light sources preferably has an equivalent angle defined by a straight line (optical path L1 in Fig. 2) connecting the light source and the irradiation position P2, and an extension line of an optical path (optical path L6 in Fig. 2) connecting the irradiation position P2 and the detector unit 20. Each adjacent light sources of the plurality of light sources preferably have an equivalent angle defined by straight lines connecting the adjacent light sources and the irradiation position P2. The plurality of light sources is preferably arranged at equivalent intervals around the extension line of the straight line (optical path L6) connecting the irradiation position P2 and the detector unit 20.

With the above-described configuration, luminance unevenness caused by the near infrared light output from the light source can be prevented. Thus, the detector unit can detect the diffuse reflection light with higher accuracy, and the quality can be further correctly evaluated.

In the food inspection apparatus according to any of the first to third embodiments, the near infrared light may be output from the light source 10 in synchronization with the detection of the diffuse reflection light by the detector unit 20. In this case, the detector unit 20 can efficiently detect only the diffuse reflection light caused by the near infrared light output from the light source 10, and hence, food inspection with higher accuracy can be carried out.

Examples
Next, a variation in spectral shape of a diffuse reflectance spectrum obtained through measurement of food with a foreign material placed thereon is described. The variation in spectral shape is caused by changing the arrangement of the light source and the detector unit.

The food inspection apparatus 1 according to the first embodiment and a food inspection apparatus, in which the angle a1 of the food inspection apparatus 1 is changed to an angle smaller than 45 degrees, were used as the food inspection apparatuses. A halogen lamp was used as the light source, and an MCT detector unit was used as the detector unit. A raisin, which is food, with a hair as a foreign material placed thereon was used as the object. A surface of food (food) and a surface of a foreign material (foreign material) placed on the food served as irradiation positions. The irradiation positions were irradiated with near infrared light in a wavelength ranging from 1000 to 2100 nm and diffuse reflectance spectra were measured.

Diffuse reflectance spectra of the food and the foreign material were measured for each of the angles a1 of 50, 60, 70, and 80 degrees as examples. Also, diffuse reflectance spectra of the food and the foreign material were measured for each of the angles a1 of 20, 30, and 40 degrees as comparative examples. The diffuse reflectance spectra obtained through the measurement were converted by Kubelka-Munk conversion (KM conversion), thereby obtaining absorption spectra, and the absorption spectra were second-differentiated, thereby obtaining KM absorbance second differentiation spectra.

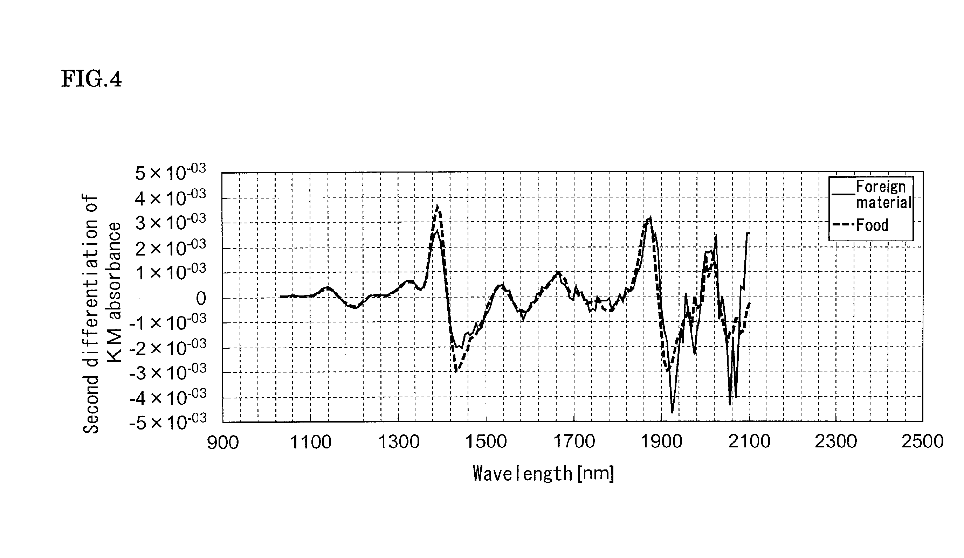

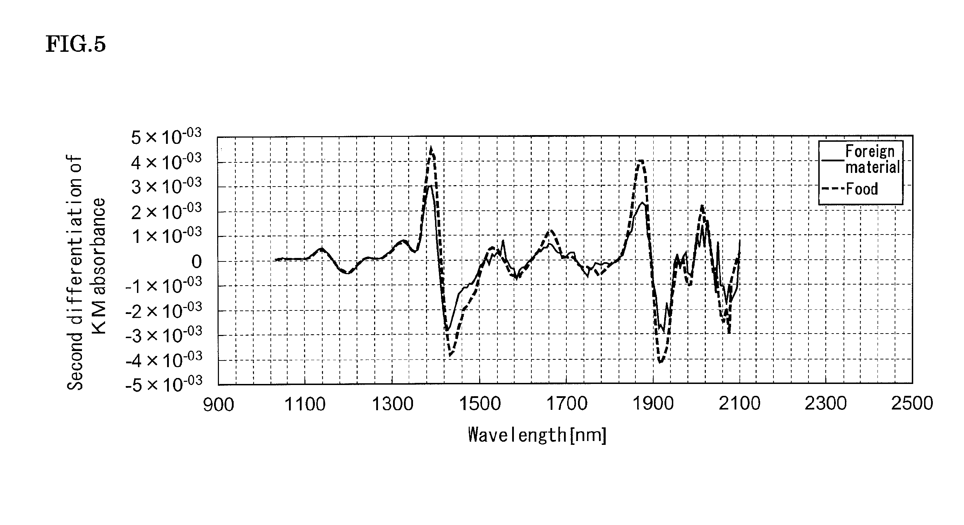

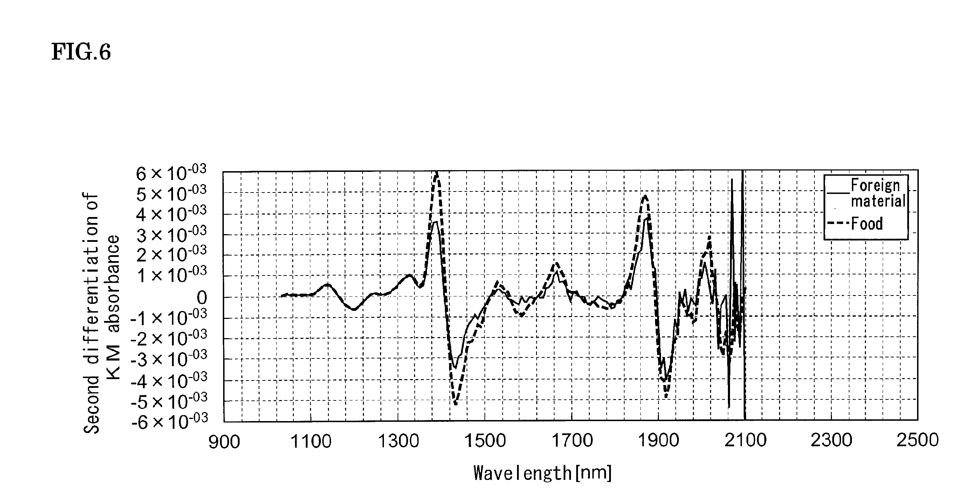

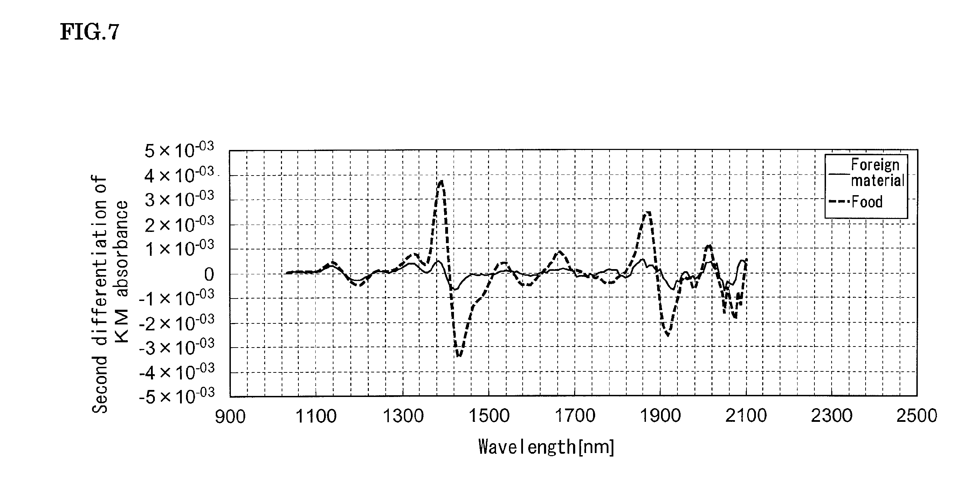

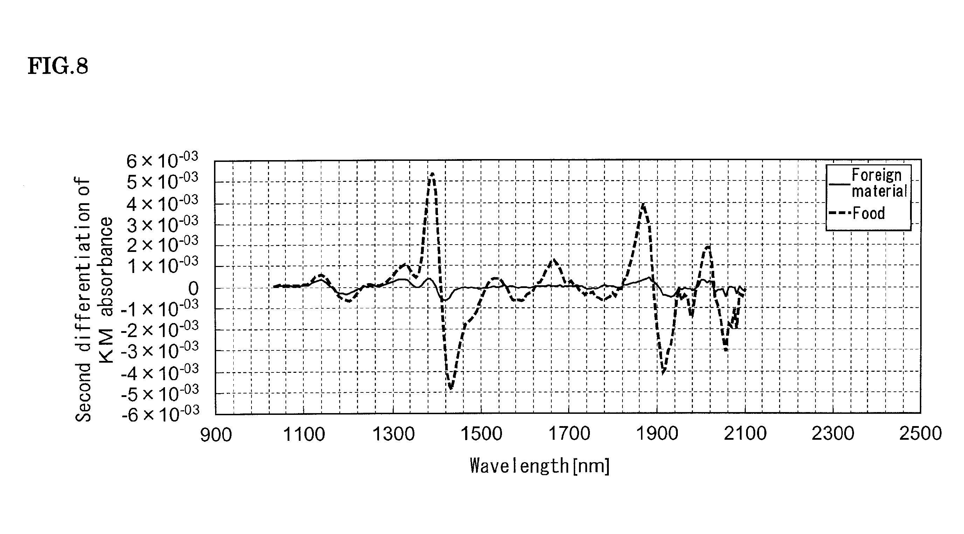

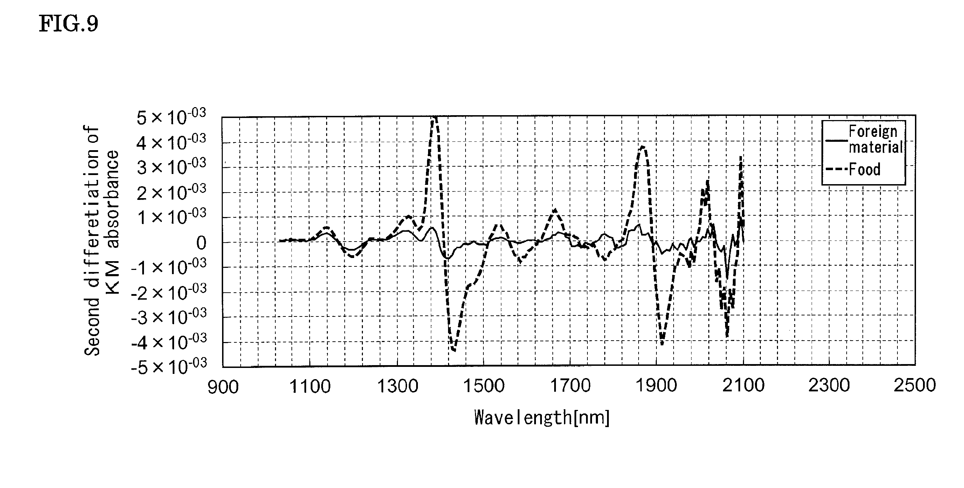

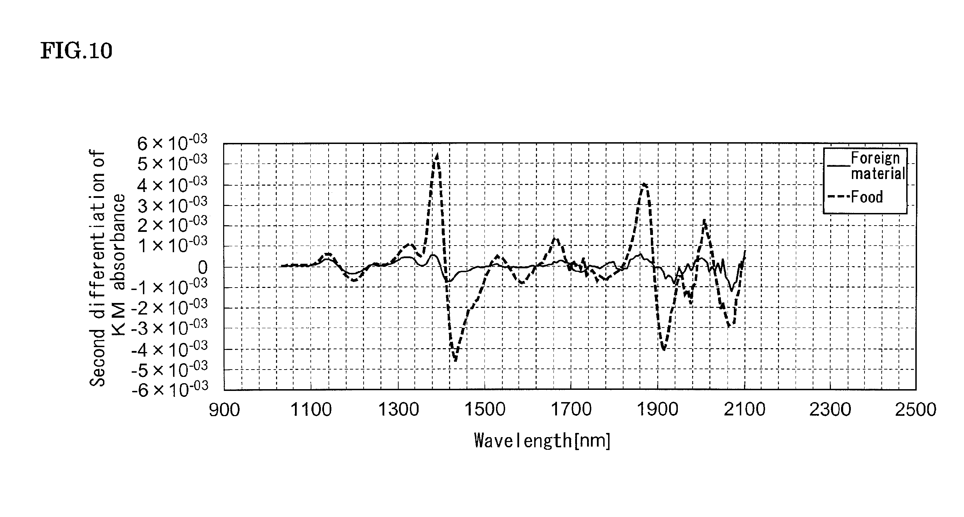

Figures 4 to 10 show the KM absorbance second differentiation spectra. Figures 4 to 10 respectively show the cases when the angles a1 are 20, 30, 40, 50, 60, 70, and 80 degrees. Comparing with the KM absorbance second differentiation spectra for the angles a1 of 20, 30, and 40 degrees, with the KM absorbance second differentiation spectra for the angles a1 of 50, 60, 70, and 80 degrees, it was found that the spectral shapes around the wavelengths of 1430 and 1930 nm of the food are different from those of the foreign material.

Regarding the KM absorbance second differentiation spectra for the angles a1 of 50, 60, 70, and 80 degrees in the case of the raisin, the spectral shapes are markedly varied around the wavelengths of 1430 and 1930 nm. Great peaks appear both in positive and negative directions. In contrast, regarding the foreign material, the spectra around the wavelengths of 1430 and 1930 nm exhibit only small variations. Accordingly, it was found that the shapes of the KM absorbance second differentiation spectra of the food are different from those of the foreign material. In contrast, regarding the KM absorbance second differentiation spectra for the angles a1 of 20, 30, and 40 degrees, the KM absorbance second differentiation spectra around the above-mentioned wavelengths of the food were not markedly different from those of the foreign material. As described above, it was found that it is difficult to recognize the presence of a foreign material as long as the light source and the detector unit were arranged such that the angle a1 is any of 20, 30, and 40 degrees.

Thus, with the above-described examples, it was found that a foreign material can be detected with high accuracy as long as the light source and the detector unit were arranged such that the angle a1 is 45 degrees or greater.

## Claims

1. A food inspection apparatus that measures diffuse reflection light obtained by irradiating food as an object with near infrared light and evaluates quality of the object on the basis of the measured result, the apparatus comprising:
a light source or a plurality of light sources that outputs the near infrared light; and
a detector unit that measures a diffuse reflectance spectrum of light which is the near infrared light output from the light source or the plurality of light sources and is diffusively reflected by the object,
wherein an angle defined by an optical path, along which the near infrared light is regularly reflected at a surface of the object, and a straight line connecting an irradiation position on the surface of the object irradiated with the near infrared light and the detector unit, is 45 degrees or greater.

2. The food inspection apparatus according to claim 1,
wherein the light source or the plurality of light sources is the plurality of light sources,
each of the plurality of light sources has an equivalent angle defined by a straight line connecting the light source and the irradiation position, and the straight line connecting the irradiation position and the detector unit, and
the plurality of light sources are arranged at equivalent intervals around the straight line connecting the irradiation position and the detector unit.

3. A food inspection apparatus that measures diffuse reflection light obtained by irradiating food as an object with near infrared light and evaluates quality of the object on the basis of the measured result, the apparatus comprising:
a light source or a plurality of light sources that outputs the near infrared light; and
a detector unit that measures a diffuse reflectance spectrum of light which is the near infrared light output from the light source or the plurality of light sources and is diffusively reflected by the object from among;
wherein an angle defined by an optical path, along which the near infrared light is transmitted through the object, and a straight line connecting an irradiation position on the surface of the object irradiated with the near infrared light and the detector unit, is 45 degrees or greater.

4. The food inspection apparatus according to claim 3,
wherein the light source or the plurality of light sources are the plurality of light sources,
each of the plurality of light sources has an equivalent angle defined by a straight line connecting the light source and the irradiation position, and an extension line of the straight line connecting the irradiation position and the detector unit, and
the plurality of light sources are arranged at equivalent intervals around the extension line of the straight line connecting the irradiation position and the detector unit.

5. The food inspection apparatus according to claim 1 or 3,
wherein an intensity of the near infrared light output from the light source or the plurality of light sources is modulated, and
the detector unit measures the diffuse reflectance spectrum of the light diffusively reflected by the object in synchronization with a cycle of the intensity modulation of the light source or the plurality of light sources from among the near infrared light output from the light source or the plurality of light sources.

6. The food inspection apparatus according to claim 1 or 3, further comprising polarizing plates arranged between the light source or the plurality of light sources and the irradiation position of the near infrared light output from the light source or the plurality of light sources, and between the irradiation position and the detector unit, the polarizing plates selectively transmitting linearly polarized light component in a direction.

7. The food inspection apparatus according to claim 1 or 3, wherein the light source or the plurality of light sources is a halogen lamp or a plurality of halogen lamps.

8. The food inspection apparatus according to claim 1 or 3, wherein the light source or the plurality of light sources is a supercontinuum light source or a plurality of supercontinuum light sources.

9. The food inspection apparatus according to claim 1 or 3, wherein the light source or the plurality of light sources is a laser light source or a plurality of laser light sources that outputs light with a wavelength ranging from 1000 to 2500 nm.
